(19) Europäisches Patentamt
European Patent Office
Office européen des brevets

(11) Numéro de publication: **0 363 273 B1**

(12) **FASCICULE DE BREVET EUROPEEN**

(45) Date de publication de fascicule du brevet: **24.11.93**

(51) Int. Cl.5: **C07D 513/22**, A61K 31/54, C07D 209/14, C07D 417/06, C07D 513/14, //(C07D513/22, 279:00,221:00,221:00,209:00), (C07D513/14,279:00,221:00, 209:00)

(21) Numéro de dépôt: **89402727.5**

(22) Date de dépôt: **03.10.89**

(54) **Dérivés 17 soufrés de la 20,21-dinoréburnaménine, leur procédé de préparation et les intermédiaires ainsi obtenus, leur application comme médicaments et les compositions pharmaceutiques les renfermant.**

(30) Priorité: **06.10.88 FR 8813107**

(43) Date de publication de la demande:
**11.04.90 Bulletin 90/15**

(45) Mention de la délivrance du brevet:
**24.11.93 Bulletin 93/47**

(84) Etats contractants désignés:
**CH DE GB IT LI NL**

(56) Documents cités:
**DE-A- 2 736 784**

(73) Titulaire: **ROUSSEL-UCLAF**
**35, Boulevard des Invalides**
**F-75007 Paris(FR)**

(72) Inventeur: **Clemence, François**
**2, rue Turgot**
**F-75009 Paris(FR)**
Inventeur: **Haesslein, Jean-Luc**
**18, Avenue du Président Kennedy**
**F-93110 Rosny sous Bois(FR)**
Inventeur: **Oberlander, Claude**
**2, rue Paul Albert**
**F-75018 Paris(FR)**

(74) Mandataire: **Bourgouin, André et al**
**Département des Brevets**
**ROUSSEL UCLAF**
**111, route de Noisy**
**B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.6/3.3.1)

## Description

L'invention concerne de nouveaux dérivés 17 soufrés de la 20,21-dinoréburnaménine, leur procédé de préparation et les nouveaux intermédiaires ainsi obtenus, leur application comme médicaments et les compositions les renfermant.

L'invention concerne les composés de formule (I) :

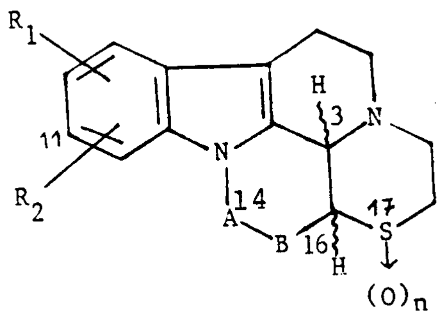

(I)

dans laquelle $R_1$ et $R_2$, identiques ou différents, représentent un atome d'hydrogène, un atome d'halogène, un radical alkyle ou alcoxy renfermant de 1 à 5 atomes de carbone, un radical hydroxy, trifluorométhyle ou nitro et dans laquelle n représente 0,1 ou 2 et le groupe :

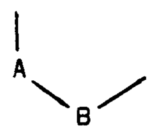

représente : soit

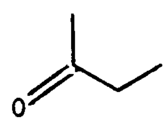

,

,soit

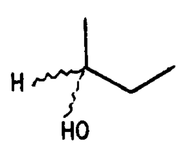

,

soit

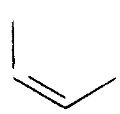

,

soit

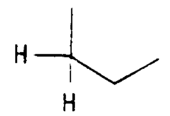

lesdits produits de formule (I) étant sous toutes les formes isomères possibles racémiques ou optiquement actives, ainsi que les sels d'addition avec les acides minéraux ou organiques desdits produits de formule (I).

Dans les produits de formule (I), l'atome d'hydrogène en position 3 et l'atome d'hydrogène en position 16, peuvent chacun occuper l'une ou l'autre dés orientations alpha et béta, ce qui détermine l'existence de diastéréoisomères cis et trans. De même, le radical hydroxy en position 14 peut être sous la forme alpha ou béta.

Lorsque $R_1$ et $R_2$ représentent un radical alkyle, il s'agit de préférence des radicaux méthyle, éthyle, n-propyle ou isopropyle mais ces substituants peuvent aussi représenter un radical n-butyle, isobutyle, n-pentyle.

Lorsque $R_1$ et $R_2$ représentent un radical alcoxy, il s'agit de préférence d'un radical méthoxy ou éthoxy, mais ils peuvent aussi représenter un radical propoxy, isopropoxy, butoxy linéaire, secondaire ou tertiaire.

Lorsque $R_1$ et $R_2$ représentent un atome d'halogène, il s'agit de préférence de l'atome de chlore, mais ils peuvent aussi représenter un atome de fluor, de brome ou d'iode.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcanemonosulfoniques, tels que l'acide méthanesulfonique, l'acide éthanesulfonique, l'acide propanesulfonique, les acides alcanedisulfoniques tels que l'acide méthanedisulfonique, l'acide alpha,béta-éthanedisulfonique, les acides arylmonosulfoniques, tel que l'acide benzènesulfonique et les acides aryldisulfoniques.

L'invention a plus particulièrement pour objet les composés de formule I tels que définis ci-dessus caractérisés en ce que $R_1$ et $R_2$ représentent tous deux un atome d'hydrogène et en ce que le groupe :

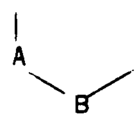

représente soit

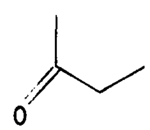

soit

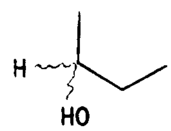

soit

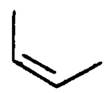

L'invention a tout particulièrement pour objet les produits décrits ci-après dans les exemples et notamment : la (±) 20,21 dinor 17-thia éburna ménine 14(15H)-one et ses sels avec les acides minéraux ou organiques notamment le fumarate, ou bien le (14alpha,16alpha) (±) 14,15-dihydro 20,21-dinor 17-thia eburnaménine-14-ol.

L'invention a aussi pour objet un procédé de préparation caractérisé en ce que l'on soumet un composé de formule (II) :

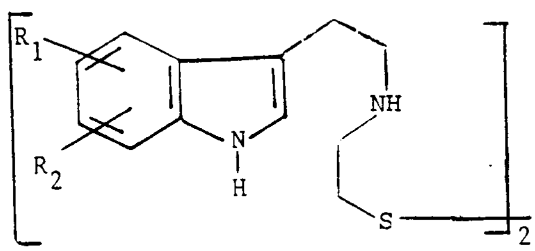

(II)

dans laquelle $R_1$ et $R_2$ ont les significations données précédemment à l'action d'un chlorure d'acide de formule (III) ou (III') :

(III') $RO_2C\text{-}CH=CH\text{-}COCl$ ,

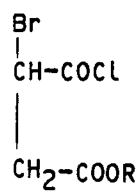

(III)

dans laquelle R représente un radical alkyle renfermant 1 ou 2 atomes de carbone pour obtenir un composé de formule (IV) :

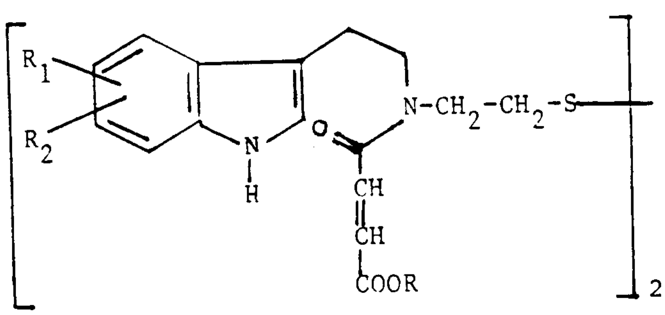

(IV)

soumet le disulfure de formule (IV) à l'action d'un agent réducteur pour obtenir un composé de formule (IV') :

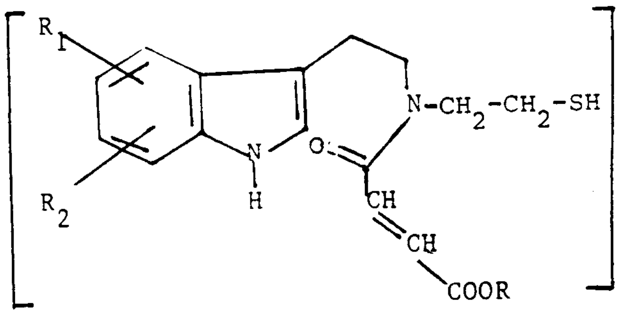

(IV')

qui se cyclise spontanément en composé de formule (V) :

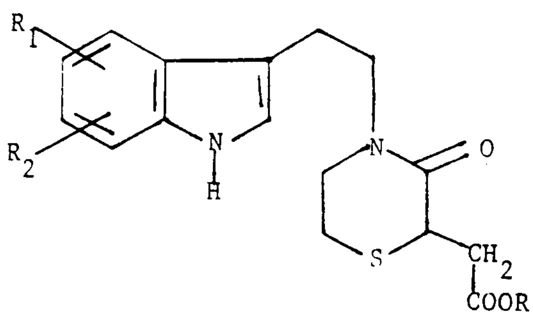

(V)

cyclise le composé de formule (V) en sel de formule (VI) :

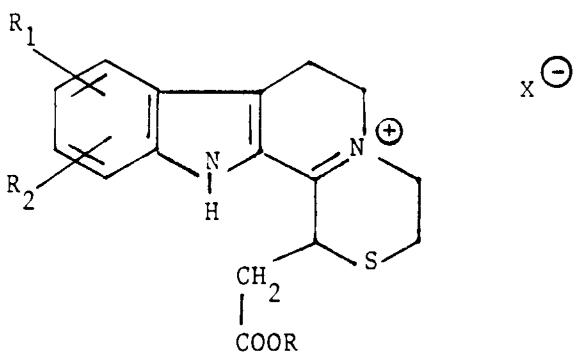

(VI)

dans laquelle X⁻ représente un contre-ion et que soit l'on réduit le sel de formule (VI) pour obtenir un composé de formule (VII) dans laquelle les deux hydrogènes sont en position cis

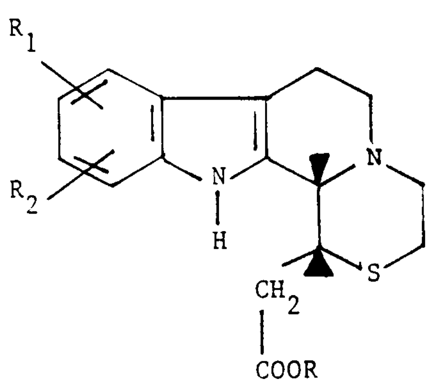

(VII)

produit de formule (VII) que l'on cyclise en milieu basique pour obtenir un composé de formule ($I_{A1}$) correspondant à un composé de formule (I) dans laquelle

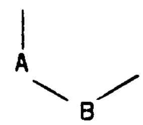

représente

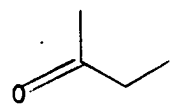

l'atome d'hydrogène en position 3 et l'atome d'hydrogène en position 16 sont en position cis l'un par rapport à l'autre, et n est égal à 0 ;
soit l'on cyclise le sel de formule VI en composé de formule (VIII) :

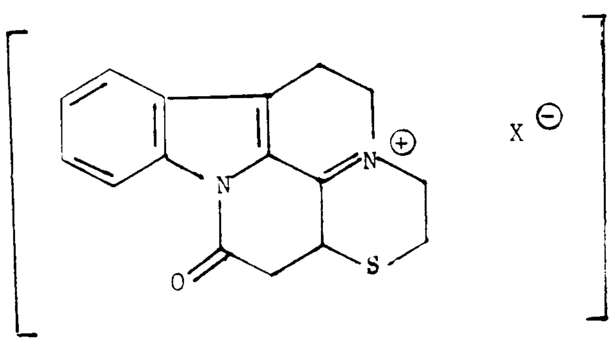

(VIII)

que l'on réduit pour obtenir un composé de formule ($I_{A2}$) correspondant à un composé de formule (I) dans laquelle

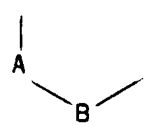

représente

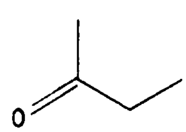

l'atome d'hydrogène en position 3 et l'atome d'hydrogène en position 16 sont en position trans l'un par rapport à l'autre et n est égal à 0 et en ce que lesdits composés de formules ($I_{A1}$) et ($I_{A2}$) sont, si désiré, réduits soit en composés correspondants de formules ($I_{B1}$) et ($I_{B2}$) représentant les composés de formule (I) dans laquelle

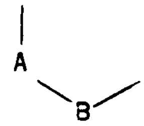

représente

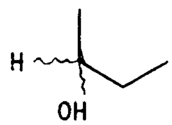

et n est égal à 0 soit en composé de formules ($I_{C1}$) et ($I_{C2}$) représentant les composés de formule (I) dans laquelle

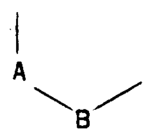

représente

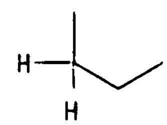

et n est égal à 0 et en ce que, si désiré, on déshydrate les composés de formules ($I_{B1}$) et ($I_{B2}$) pour obtenir les composés de formule ($I_{D1}$) et ($I_{D2}$) représentant les composés de formule (I) dans laquelle

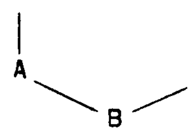

représente

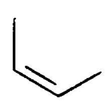

et n est égal à 0, et en ce que l'on soumet si désiré les composés de formules ($I_{A1}$), ($I_{A2}$), ($I_{B1}$), ($I_{B2}$), ($I_{C1}$), ($I_{C2}$), ($I_{D1}$) et ($I_{D2}$) à l'action d'un agent d'oxydation pour obtenir les composés correspondants dans lesquels n représente 1 ou 2 et, si désiré, soumet tous les produits de formule (I) obtenus à l'action d'un acide minéral ou organique pour obtenir le sel correspondant.

Dans les conditions préférentielles de mise en oeuvre de l'invention, le procédé ci-dessus est réalisé de la manière suivante :

- R est de préférence un radical méthyle dans le composé de formule (III).
- Pour obtenir le composé de formule (IV), on opère en présence d'une base comme le carbonate de potassium, la soude ou la potasse et d'un agent de transfert de phase, notamment le bromure ou l'hydrogénosulfate de tétrabutyl ammonium.

- L'agent de réduction du disulfure de formule (IV) est la triphénylphosphine. On opère en présence d'un mélange monoglyme/eau ou d'un mélange diglyme, tétrahydrofuranne ou dioxanne/eau.
- La cyclisation du composé de formule (V) en composé de formule (VI) s'effectue par l'oxychlorure de phosphore. $X^{\ominus}$ représente donc un ion chlorure.
- La réduction du composé de formule (VI) en composé de formule (VII) s'effectue avec du borohydrure de sodium en présence de méthanol.
- La cyclisation en milieu basique du composé de formule (VII) est réalisée par un mélange alcoolate/alcool correspondant tel que par exemple le mélange éthanol/éthylate de sodium ou méthanol/méthylate de sodium.
- La cyclisation du composé de formule (VI) en composé de formule (VIII) s'effectue par l'acide chlorhydrique et la réduction du composé de formule (VIII) par le borohydrure de sodium en présence d'acide acétique.
- La réduction des composés de formules ($I_{A1}$) ou ($I_{A2}$) en composés de formules ($I_{B1}$) ou ($I_{B2}$) ou ($I_{C1}$) ou ($I_{C2}$) est réalisée avec un hydrure ou un hydrure mixte, tel que par exemple, l'hydrure mixte de lithium et d'aluminium, le diéthylhydrure de sodium et d'aluminium, l'hydrure de diisobutylaluminium ou le borohydrure de sodium. Pour obtenir un composé de formule ($I_{B1}$) ou($I_{B2}$)on opère à 0¤C alors que pour obtenir un composé de formule ($I_{C1}$) ou ($I_{C2}$) on opère plutôt à température ambiante.
- L'agent de déshydratation utilisé pour obtenir à partir des composés de formules ($I_{B1}$) et ($I_{B2}$) dans lesquelles

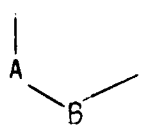

représente

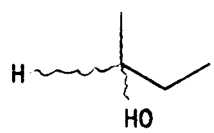

les composés de formules ($I_{D1}$) et ($I_{D2}$) dans lesquelles

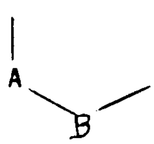

représente

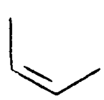

est un acide tel que l'acide chlorhydrique, l'acide bromhydrique, l'acide sulfurique, l'acide acétique, l'acide paratoluènesulfonique, l'acide méthanesulfonique en quantité catalytique.
- Pour oxyder les composés de formule (I) dans laquelle n = 0 en composés de formule (I) dans laquelle n = 1 ou 2 on utilise l'acide méta-chloroperbenzoïque, le monoperphtalate de magnésium ou le périodate de sodium.
- Les formes optiquement actives des produits de formule (I) peuvent être préparées par dédoublement des racémiques selon les méthodes usuelles. L'invention concerne aussi une variante du procédé de préparation pour obtenir les composés de formule (I) dans laquelle n est égal à 1 ou 2, caractérisé en ce que l'on soumetles produits de formules ($I_{A1}$) et ($I_{A2}$) dans lesquelles n est égal à 0 à l'action d'un

agent d'oxydation pour obtenir des produits correspondants dans lesquels n est égal à 1 ou à 2 produits que l'on réduit, si désiré, en composés correspondants dans lesquels n est égal à 1 ou 2 et

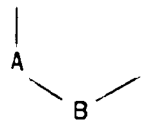

représente

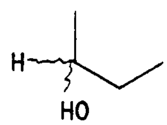

ou

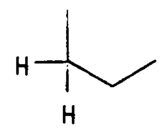

et en ce que, si désiré, l'on déshydrate les produits ainsi obtenus dans lesquels

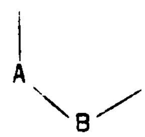

représente

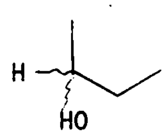

pour obtenir les composés correspondants dans lesquels n est égal à 1 ou 2 et

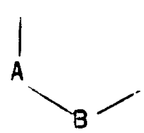

représente

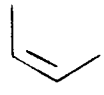

Dans cette variante les transformations des composés correspondants aux composés de formules ($I_{A1}$) et ($I_{A2}$) dans lesquels n représente 1 ou 2 est effectuée dans les mêmes conditions que les transformations correspondantes dans la série des produits où $n = 0$.

Les composés de formule (I) tels que définis ci-dessus ainsi que leurs sels d'addition avec les acides présentent d'intéressantes propriétés pharmacologiques.

Ils possèdent des propriétés analgésiques et également des propriétés protectrices neuronales anti-anoxiques et anti-ischémiques intéressantes.

Certains produits peuvent aussi présenter d'intéressantes propriétés nootropes (effet anti-amnésiant et réversion d'un déficit mnésique après lésion d'un cholinergique septale), anti-dépressives, protectrices neuronales, anti-anoxiques, anti-ischémiques.

Ces propriétés justifient leur application en thérapeutique et l'invention a également pour objet à titre de médicaments, les produits tels que définis par la formule (I) ci-dessus, lesdits produits de formule (I) étant sous toutes les formes isomères possibles racémiques ou optiquement actives ainsique les sels d'addition avec les acides minéraux ou organiques pharmaceutiquement acceptables desdits produits de formule (I).

L'invention a plus particulièrement pour objet, à titre de médicaments, les composés de formule (I) tels que définis ci-dessus caractérisés en ce que $R_1$ et $R_2$ représentent tous deux un atome d'hydrogène et en ce que le groupement

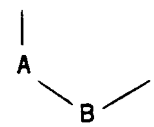

représente soit

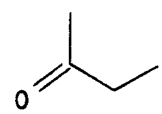

soit

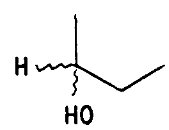

soit

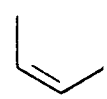

L'invention a tout particulièrement pour objet, à titre de médicaments les composés de formule (I) répondant aux formules suivantes :

- la (±) 20,21 dinor 17-thia éburnaménine-14(15H)-one,
- lLe (14alpha,16alpha) (±) 14,15-dihydro 20,21-dinor 17-thia éburnaménine-14-ol,

et leurs sels pharmaceutiquement acceptables.

Les médicaments, objet de l'invention, peuvent être utilisés dans le traitement des algies musculaires, articulaires ou nerveuses, des douleurs dentaires, des migraines, du zona et également à titre de traitement complémentaire dans les états infectieux et fébriles.

Ils peuvent être aussi utilisés dans le traitement des insuffisances cérébrales d'origine anoxique ou ischémique, des troubles de la mémoire et de l'attention. Ils peuvent être également ûtilisés comme anti-dépresseurs.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que

le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple de 10 mg à 2 g par jour chez l'adulte, par voie orale.

La présente invention a enfin pour objet à titre de produits industriels nouveaux, notamment à titre de produits intermédiaires nécessaires à la préparation des produits de formule (I), les produits de formules (IV), (IV'), (V), (VI), (VII) et (VIII).

Certains des produits de formule (II) utilisés au départ de l'invention sont connus (voir par exemple la publication soviétique Tr. Mosk. Khim Tekhnol. Inst. im. D.I. Mendeleeva (1977), 94, 9-19 rapportée aux Chemical Abstracts 92 128821.

Les produits de formule (II) qui ne sont pas connus peuvent par exemple être préparés par le procédé suivant.

On fait agir le chlorure de chloroacétyle sur un dérivé de tryptamine de formule (B) :

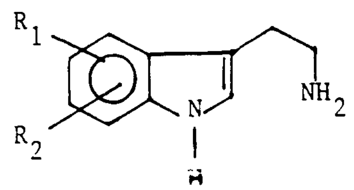

(B)

dans laquelle $R_1$ et $R_2$ ont la signification précédente pour obtenir un produit de formule (C) :

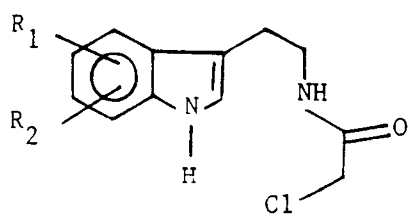

(C)

produit de formule (C) sur lequel on fait agir du thiosulfate de sodium pour obtenir un produit de formule (D) :

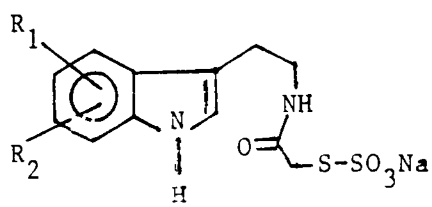

(D)

que l'on réduit, par exemple par l'hydrure de lithium aluminium pour obtenir le produit de formule (II) recherché.

On peut également indiquer que certains des produits de formule (D) sont connus (voir par exemple la publication soviétique Khim. Geterotsikl. Soedin. (11) 1505-11 1973 rapportée aux Chemical Abstracts 80, 82567. Il en est de même de certains des produits de formule (C) (voir les publications suivantes) :

Tetrah. Lett. 1975 (39) 3399-402
Chem. Pharm. Bull 28(3), 900-9 (1980)
Tetrah. Lett. 26(37), 4443-6 (1985)
J. Med. Chem. 12(4) 636.8 (1969)
Izv. Akad. Nauk. Arm. SSR. 14, 603, 10 (1961)
Yakugaku Zasshi 81, 636-9 (1961)
Rev. Chim. (Bucharest) 19(8) 444-7 (1968)
Khim. Geterotsikl. Soedin. (11) 1505-11 (1973)

J. Pharm. Pharmacol. 31(6), 371-4 (1979).

Les exemples suivants illustrent l'invention sans toutefois la limiter.

**Exemple 1 : (±)-20,21-dinor 17-thia éburnaménine-14(15H)-one.**

**Stade A :** 5,5'-bis[2-(3-indolyl) éthyl] 4,4'-dioxo 7,7' dithio di(5-aza 2-heptènoate de méthyle).

a) Préparation du chlorure d'acide

On ajoute 7,5 $cm^3$ de chlorure de thionyle dans une solution de 6,77 g de 2-bromo succinate acide de (4) méthyle 67 $cm^3$ de chloroforme et chauffe au reflux pendant une heure. On distille le chloroforme et l'excès de chlorure de thionyle sous pression réduite. On récupère 7 g de chlorure d'acide utilisé brut ultérieurement.

b) Amidification

A une solution de 6,4 g de N,N'-bis[2-(3-indolyl) éthyl] 2,2'-dithio diéthanamine dans 50 $cm^3$ de chloroforme on ajoute 20 g de carbonate de potassium dans 60 $cm^3$ d'eau puis 0,32 g de bromure de tétrabutylammonium.

Le chlorure d'acide décrit ci-dessus mis en solution dans 50 $cm^3$ de chloroforme est introduit en 5 minutes sous forte agitation. On agite ensuite pendant une nuit à température ambiante.

On décante, réextrait la phase aqueuse par deux fois 50 $cm^3$ de chloroforme et lave la phase organique par trois fois 50 $cm^3$ d'eau qui sont réextraits par 50 $cm^3$ de chloroforme. Après séchage de la phase organique et concentration sous pression réduite on obtient 11 g d'une huile que l'on chromatographie sur silice (éluant : acétate d'éthyle-chlorure de méthylène 8:2). On obtient ainsi 7,3 g de produit sous forme d'huile (Rf = 0,75).

Infra-Rouge ($CHCl_3$) $cm^{-1}$
NH indole : 3478
>C=O ester : 1724
>C=O amide : 1622
Pas de bande SH.

**Stade B** : 4-[2-(3-indolyl) éthyl] 3-oxo 2-thiomorpholineacétate de méthyle.

On ajoute 70 $cm^3$ d'eau à une solution de 7,3 g de produit obtenu au stade A dans 140 $cm^3$ de monoglyme puis introduit en une fois 3,47 g de triphénylphosphine et agite 48 heures à température ambiante.

On ajoute ensuite 100 $cm^3$ d'acétate d'éthyle et 50 $cm^3$ d'eau puis réextrait la phase aqueuse par 100 $cm^3$ d'acétate d'éthyle. On lave la solution organique totale par 50 $cm^3$ d'eau, sèche sur sulfate de sodium et concentre sous pression réduite.

On obtient 11 g d'une huile que l'on chromatographie sur silice (éluant : acétate d'éthyle-chlorure de méthylène 8:2). On obtient 6,6 g de produit pur F = 120¤C.

IR ($CHCl_3$) $cm^{-1}$
>C=O ester : 1737 NH indole : 3479
>C=O amide : 1657

**Stade C** : [1alpha,12b béta(±)]-3,4,6,7,12,12b-hexahydro 1H-[1,4-thiazino] [4',3',1,2]pyrido[3,4-b]indole-1-acétate de méthyle.

On dissout 1 g de produit préparé au stade B dans 10 $cm^3$ d'oxychlorure de phosphore chauffé à 80¤C et maintient à cette température pendant 3 heures.

On concentre le milieu réactionnel sous pression réduite et dissout l'extrait sec dans 50 $cm^3$ de méthanol puis fait agir 1 g de borohydrure de sodium à une température de 15¤ ± 5¤C. On concentre la suspension sous pression réduite puis la reprend par 10 $cm^3$ d'acétate d'éthyle et 50 $cm^3$ d'eau. On décante la phase aqueuse et réextrait par 50 $cm^3$ d'acétate d'éthyle. La totalité de la phase organique est lavée par 3 fois 25 $cm^3$ d'eau que l'on réextrait à l'acétate d'éthyle. On sèche sur sulfate de sodium. On concentre à sec. On obtient 0,78 g de solide que l'on chromatographie sur silice (éluant : chlorure de méthylène-acétate d'éthyle 8:2). On obtient 0,51 g de produit pur. F = 212¤C.

Infra-Rouge ($CHCl_3$) $cm^{-1}$
Pas de C=O lactame

NH indole : 3464
>C = O ester : 1727.

**Stade D :** (±)-20,21-dinor 17-thia éburnaménine-14(15H)-one.

On ajoute à température ambiante et sous azote 0,4 g de méthylate de sodium à une suspension de 1,2 g de produit préparé au stade C dans 48 $cm^3$ de méthanol et chauffe au reflux pendant 7 heures.

On distille le méthanol sous pression réduite, reprend par un mélange chloroforme-méthanol (2-1), concentre le filtrat sous pression réduite et récupère 0,9 g de solide que l'on purifie sur silice éluant chlorure de méthylène-méthanol (95-5).

On obtient 0,65 g de produit attendu F = 220¤C.
Infra-Rouge ($CHCl_3$) $cm^{-1}$
Absence de NH
>C = O : 1705
C = C : 1636.

**Exemple 2 : fumarate neutre de (±)-20,21-dinor 17-thia éburnaménine-14(15H)-one.**

On dissout 0,71 g de produit obtenu à l'exemple 1 dans 90 $cm^3$ d'éthanol bouillant et ajoute 0,29 g d'acide fumarique en solution dans 10 cm3 d'éthanol bouillant. On refroidit à 0¤C tout en maintenant l'agitation pendant 30 minutes. On essore le produit obtenu, le lave à l'éthanol et le sèche sous pression réduite à 50¤C. On obtient 0,71 g de produit attendu F 250¤C.

| Analyse $C_{18}$ $H_{18}$ $N_2$ $O_3$ S. M = 342,419 | | | | | | | | |
|---|---|---|---|---|---|---|---|---|
| Calculé<br>Trouvé | C% : | 63,14<br>63,1 | H% : | 5,30<br>5,20 | N% : | 8,18<br>8,2 | S% : | 9,36<br>9,4 |

**Exemple 3 : [16alpha (±)]-20,21-dinor 17-thia éburnaménine-14(15H)-one.**

On chauffe pendant 4 heures à 80¤C une solution de 6,5 g de produit obtenu au stade B de l'exemple 1 ci-dessus dans 65 $cm^3$ d'oxychlorure de phosphore. On concentre sous pression réduite et le solide obtenu est repris par 65 $cm^3$ d'acide chlorhydrique 5N puis chauffé une nuit à 80¤C. On ajoute 65 $cm^3$ d'eau à la suspension, refroidit et agite 30 minutes à 0¤C puis essore et lave le sel formé à l'eau. Le produit ainsi obtenu est remis en suspension dans 65 $cm^3$ d'acide acétique puis soumis à l'action de 5,9 g de borohydrure de sodium à 15¤C ± 5¤C.

Après 30 minutes d'agitation, on ajoute 130 $cm^3$ d'eau et neutralise par de l'ammoniaque 22¤Bé.

On essore la suspension, la lave et la sèche sous pression réduite pour obtenir 4,75 g de solide que l'on chromatographie sur silice (éluant : chlorure de méthylène-acétate d'éthyle 8:2). On obtient 3,36 g de produit attendu F = 180¤C Infra-Rouge ($CHCl_3$) $cm^{-1}$
Absence de NH
>C = O : 1709
>C = C : 1649.

**Exemple 4 : chlorhydrate de [16alpha (±)]-20,21-dinor 17-thia éburnaménine-14(15H)-one**.

On dissout 2,84 g de produit obtenu à l'exemple 3 dans 400 $cm^3$ de méthanol bouillant. On ajoute à chaud 1 $cm^3$ d'acide chlorhydrique pur 22¤Bé et laisse revenir à température ambiante. On maintient l'agitation pendant 1 heure à -10¤C puis essore, sèche et obtient 2,57 g de chlorhydrate attendu F = 230 ° C.

| Analyse $C_{16}$ $H_{16}$ $N_2$ OS, HCl. M = 320,843 | | | | | | | | | | |
|---|---|---|---|---|---|---|---|---|---|---|
| Calculé<br>Trouvé | C% : | 59,9<br>59,7 | H% : | 5,34<br>5,3 | N% : | 8,73<br>8,6 | S% : | 9,99<br>9,8 | Cl% : | 11,05<br>11,2 |

**Exemple 5 : [14béta,16alpha (±)]-14,15-dihydro 20,21-dinor 17-thia éburnaménine 14-ol.**

On dissout partiellement 4,1 g de produit obtenu à l'exemple 3 dans 80 $cm^3$ de tétrahydrofuranne, ajoute en 15 minutes à 0-5 ¤C 30,5 $cm^3$ de diéthyl dihydro aluminate de sodium dans le toluène à 0,236 moles pour 100 cm3 et agite 1 heure à 0¤C puis ajoute 40 cm3 de tétrahydrofuranne à 20 % d'eau, agite une heure à température ambiante puis essore le précipité formé et le lave au tétrahydrofuranne. On concentre la solution organique sous pression réduite et récupère 4,5 g de produit qui est un mélange des isomères 14alpha et 14béta.

Après chromatographie sur silice (éluant : chlorure de méthylène-acétone (1-1), on récupère 1,5 g de produit attendu F = 220¤C Rf = 0,6.

On obtient également 1,23 g de produit 14alpha (Rf = 0,5) et 0,88 g de mélange 14alpha et 14béta.

RMN (DMSO,ppm) (14béta)
>CH-OH : 5,58 (dd : J = 5,5 et 9)
OH : 6,61 (d, J = 9)
$H_5$ et $H_6$ : 7,06
$H_4$ et $H_7$ : 7,40(d) et 7,65(d)
Autres protons : 1,8 à 3,2.

**Exemple 6 : [14alpha,16alpha (±)-14,15-dihydro 20,21-dinor 17-thia éburnaménine-14-ol.**

On met en suspension les 0,88 g de mélange 14alpha et 14béta obtenu à l'exemple 5 dans 10 $cm^3$ de méthanol et 10 $cm^3$ de soude 5N puis chauffe une nuit au reflux. On ajoute 20 $cm^3$ d'eau glacée, agite 15 minutes à 0-5¤C puis essore et lave à l'eau. On obtient 0,800 g de produit attendu ce qui fait avec les 1,23 g de produit obtenus à l'exemple 5 un total de 2,03 g de produit 14alpha F = 265¤C Rf = 0,5 chlorure de méthylène-acétone 1:1

RMN (DMSO,ppm) 14alpha
>CH-OH : 5,94
OH : 6,33 (d)
$H_5$ et $H_6$ : 7,02 (t) et 7,09 (t)
$H_4$ et $H_7$ : 7,37 (d) et 7,44 (d)
Autres protons : 1,9 à 3,4

**Exemple 7 : Maléate acide du [16alpha (±)]-20,21-dinor 17-thia éburnaménine.**

On ajoute 0,14 g d'acide para-toluènesulfonique à une suspension de 2,8 g de produit constitué par un mélange 14alpha et 14béta tels qu'obtenu à l'exemple 5 ci-dessus dans 60 $cm^3$ de toluène séché et chauffe une nuit au reflux. On ajoute 60 $cm^3$ d'acétate d'éthyle et 60 $cm^3$ d'eau puis amène à pH alcalin par de l'ammoniaque à 22¤Bé. On filtre l'insoluble, décante réextrait la phase aqueuse à l'acétate d'éthyle, lave la solution organique à l'eau, sèche et concentre sous pression réduite. On récupère 2 g de produit que l'on chromatographie sur silice éluant chlorure de méthylène-méthanol 95:5. On obtient 1,28 g de produit sous forme de base libre F = 188¤C.

Salification :

Les 1,28 g de produit obtenu ci-dessus sont dissous dans l'acétate d'éthyle bouillant et on ajoute 0,55 g d'acide maléïque solubilisé dans 10 $cm^3$ d'acétate d'éthyle chaud. On agite la solution une heure à 0-5¤C, essore le sel qui a cristallisé et le sèche sous pression réduite. On obtient 1,67 g de maléate F = 174¤C.

Infra-Rouge ($CHCl_3$) $cm^{-1}$ (sur la base libre)
Absence d'OH
-C = C- : 1648
Aromatiques : 1615,1565

**Exemple 8 : Maléate acide du [16alpha (±)]-14,15-dihydro 20,21-dinor 17-thia éburnaménine.**

On ajoute en une seule fois sous azote et à température ambiante 30 $cm^3$ d'hydrure de diisobutylaluminium à 1 mole/l dans l'hexane à une solution de 1,5 g de produit obtenu à l'exemple 3 dans 30 $cm^3$ de tétrahydrofuranne sec. On agite 4 heures à température ambiante. On ajoute 15 $cm^3$ d'un mélange tétrahydrofuranne-eau 90:10. On agite 15 minutes à 0¤C filtre la suspension, lave le précipité avec un

mélange chloroforme-méthanol 2:1. On évapore à sec et chromatographie le résidu sur silice (éluant : chlorure de méthylène-acétone 95:5). On obtient 0,7 g de cristaux blancs de la base libre F = 138¤C.
Infra-Rouge ($CHCl_3$) $cm^{-1}$
Absence de >C = O
Aromatiques ) 1618
-C = C- ( 1480

Salification :

On ajoute 0,128 g d'acide maléique à une solution de 300 mg de base obtenue ci-dessus dans 10 $cm^3$ d'un mélange acétate d'éthyle-éthanol 1:1. On agite 1 heure 30 à température ambiante, filtre, lave le précipité avec un mélange acétate d'éthyle-éthanol 1:1, puis sèche. On obtient 300 mg de produit salifié sous forme de cristaux F = 164¤C.

**Exemple 9 : 17-oxyde de [16alpha (±)]-20,21-dinor 17-thia éburnaménine-14(15H)-one.**

On ajoute 1,02 g de perphtalate de magnésium hydraté à 6 molécules d'eau en solution dans 150 $cm^3$ d'eau dans une solution de 1 g de produit obtenu à l'exemple 3 dans 60 $cm^3$ d'éthanol chaud. On chauffe 2 heures à 50¤C. On évapore l'éthanol, alcalinise la phase aqueuse avec du bicarbonate de sodium. On extrait au chloroforme le produit précipité et obtient 900 mg de produit brut attendu que l'on chromatographie sur silice éluant chlorure de méthylène-méthanol 9:1. On obtient 690 mg de produit attendu F = 240¤C.
Infra-Rouge ($CHCl_3$,$cm^{-1}$)
>C = O : 1709
-C = C : 1649
-S O : 1058.

**Exemple 10 : Maléate du 17-oxyde de [16alpha (±)] 20,21-dinor 17-thia éburnaménine-14(15H)-one.**

On solubilise à chaud 0,9 g de base obtenue à l'exemple 9 dans un mélange de 50 $cm^3$ d'éthanol et 75 $cm^3$ d'acétate d'éthyle. On ajoute 0,347 g d'acide maléïque en solution dans 25 $cm^3$ d'acétate d'éthyle chaud. On agite 20 minutes et le sel précipite. On agite 2 heures à température ambiante filtre et sèche sous pression réduite. On obtient 0,95 g de cristaux blancs F = 216¤C.

| Analyse : $C_{16}H_{16}N_2O_2S$, $C_4H_4O_4$ M = 416,455 | | | | | | | | |
|---|---|---|---|---|---|---|---|---|
| Calculé<br>Trouvé | C%. : | 57,68<br>57,9 | H% : | 4,84<br>4,8 | N% : | 6,72<br>6,7 | S% : | 7,83<br>7,6 |

**Exemple 11 : 17-oxyde de [14alpha,16alpha (±)] 14,15-dihydro 20,21-dinor 17-thia éburnaménine-14-ol et 17-oxyde de [14béta,16alpha (+)] 14,15-dihydro 20,21-dinor 17-thia éburnaménine-14-ol.**

On ajoute par fractions 0,7 g de borohydrure de sodium à une solution de 1,4 g de produit obtenu à l'exemple 9 dans 200 $cm^3$ de méthanol à 10 % aqueux.

On chauffe 4 heures au reflux puis ajoute de nouveau 0,7 g de borohydrure de sodium. On agite une heure au reflux, évapore le solvant et reprend le résidu par 50 $cm^3$ de chlorure de méthylène. On lave la solution organique avec 20 $cm^3$ d'eau puis sèche sur sulfate de sodium. On obtient 1,4 g de cristaux F = 260¤C sous forme de mélange 14alpha et 14béta.

On sépare les deux isomères par trois chromatographies successives sous faible pression éluant chlorure de méthylène-méthanol 95:5.

On obtient 3 lots : 200 puis 400 mg de produit 14alpha et 600 mg de produit 14béta.

On recristallise les deux premiers lots et obtient 0,328 g de produit pur F > 260¤C (14alpha).
RMN (DMSO,ppm)

14alpha : OH : 6,46 (d,J=6Hz)

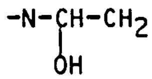 : 5,65

14béta : OH : 6,68 (d,J=9Hz)

6,8 (d,J=9Hz)

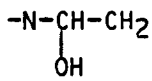 : 6,06 ppm

**Example 12 : 17-oxyde de [16alpha (+)] 20,21-dinor 17-thia éburnaménine**.

On ajoute 0,066 g d'acide para-toluène sulfonique à une suspension de 1,33 g de mélange d'isomères 14alpha et 14béta obtenu à l'exemple 11 et chauffe pendant 15 heures au reflux. On refroidit la solution à 0¤C et alcalinise avec de l'ammoniaque 22¤Bé. On extrait trois fois la phase aqueuse avec 60 $cm^3$ d'acétate d'éthyle, lave la phase organique avec 30 $cm^3$ d'eau et sèche sur sulfate de sodium. Après évaporation du solvant, on obtient 1,8 g de produit que l'on chromatographie sur silice éluant chlorure de méthylène-méthanol 95:5. On obtient ainsi 0,95 g de cristaux F = 220¤C.
RMN ($CDCl_3$,ppm)

3,65 (ddd J=12-2,5-2Hz ) H angulaire

4,39 (dl J=12Hz) ( )

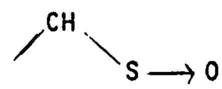

5,29 (dd J=8 et 2Hz)

5,59 (dd J=8 et 2Hz)

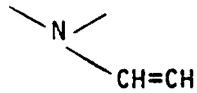

**Exemple 13 : Maléate du 17-oxyde de [16alpha (±)] 20,21-dinor 17-thia éburnaménine**.

0,9 g de base obtenue à l'exemple 12 sont mis en solution dans 180 $cm^3$ d'acétate d'éthyle chaud. On filtre la solution chaude et ajoute 0,387 g d'acide maléïque en solution dans 4 $cm^3$ d'acétate d'éthyle chaud. Après 15 minutes d'agitation à température ambiante le sel cristallise. On agite 3 heures à température ambiante. On filtre les cristaux les lave à l'acétate d'éthyle et sèche sous pression réduite à 60¤C.

On obtient 0,85 g de sel attendu F = 178¤C.
RMN (DMSO,ppm)

5,36 (dd, J=8 et 2Hz) ) >N—CH=CH-
5,48 (dd, J=8 et 2Hz) (

4,42 (dl, J=12Hz) = 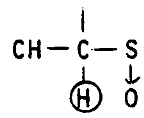

7,10 (t) ) $H_5$ et $H_6$ indole
7,20 (t) (

7,48 (d) ) $H_4$ et $H_7$ indole
7,63 (d) (

**Exemple 14 : composition pharmaceutique.**

On a préparé des comprimés répondant à la formule suivante :

- Produit de l'exemple 6 50 mg
- Excipient pour un comprimé terminé à 350 mg

(détail de l'excipient : lactose, talc, amidon, stéarate de magnésium).

**Etude pharmacologique des produits de l'invention**

Test de l'anoxie hypobare chez la Souris

Des lots de 10 souris sont placées dans une enceinte de 2 litres dans laquelle on réalise en 55 secondes une dépression de 620 mmHg. Le temps de survie est mesuré à partir du temps TO et sur une durée maximale de 3 minutes.

Les produits sont administrés par voie i.p. à la dose de 10 mg/kg et sous un volume de 10 ml/kg, 60 minutes avant l'épreuve.

Les résultats suivants, exprimés en pourcentage d'augmentation de la durée de survie, ont été obtenus :

Produit de l'exemple 2 : + 16 %
Produit de l'exemple 6 : + 20 %

## Revendications

**1.** Composés de formule (I) :

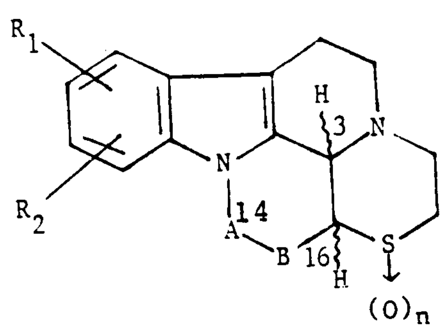

(I)

dans laquelle $R_1$ et $R_2$, identiques ou différents, représentent un atome d'hydrogène, un atome d'halogène, un radical alkyle ou alcoxy renfermant de 1 à 5 atomes de carbone, un radical hydroxy, trifluorométhyle ou nitro et dans laquelle n représente 0,1 ou 2 et le groupe :

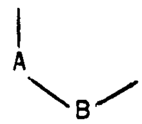

représente : soit

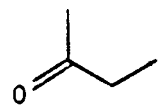

,

soit

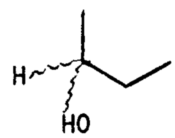

,

soit

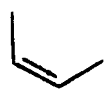

,

soit

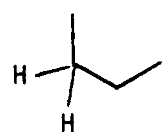

,

lesdits produits de formule (I) étant sous toutes les formes isomères possibles racémiques ou optiquement actives, ainsi que les sels d'addition avec les acides minéraux ou organiques desdits produits de formule (I).

**2.** Composés de formule (I) tels que définis à la revendication 4, caractérisés en ce que $R_1$ et $R_2$ représentent tous deux un atome d'hydrogène et en ce que le groupe :

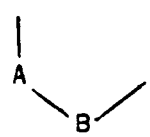

représente soit

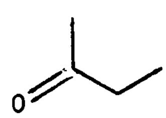

soit

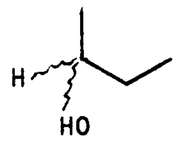

soit

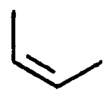

3. Composés de formule (I) telle que définie à l'une des revendications 1 ou 2 et répondant aux formules suivantes :
   - la (±) 20,21 dinor 17-thia éburnaménine 14(15H)-one et ses sels avec les acides minéraux ou organiques notamment le fumarate,
   - le (14alpha,16alpha) (±) 14,15-dihydro 20,21-dinor 17-thia eburnaménine-14-ol.

4. Procédé de préparation des composés de formule (I) tels que définis à la revendication 1, caractérisé en ce que l'on soumet un composé de formule (II) :

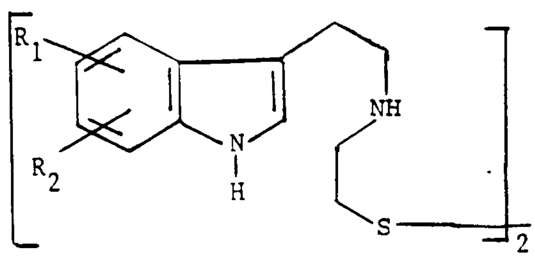

(II)

dans laquelle $R_1$ et $R_2$ ont les significations données précédemment à l'action d'un chlorure d'acide de formule (III) ou (III') :

(III') $RO_2C-CH=CH-COCl$ ,

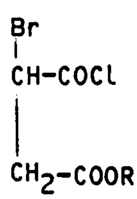

(III)

dans laquelle R représente un radical alkyle renfermant 1 ou 2 atomes de carbone pour obtenir un composé de formule (IV) :

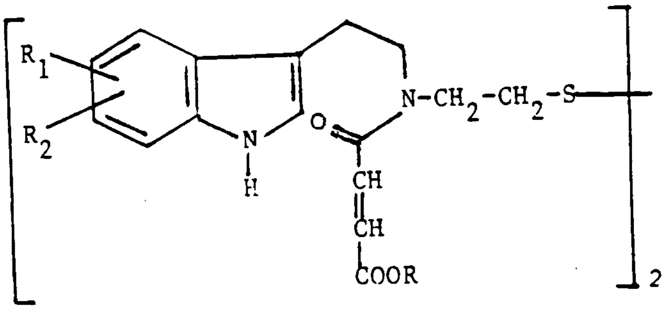

(IV)

soumet le disulfure de formule (IV) à l'action d'un agent réducteur pour obtenir un composé de formule (IV') :

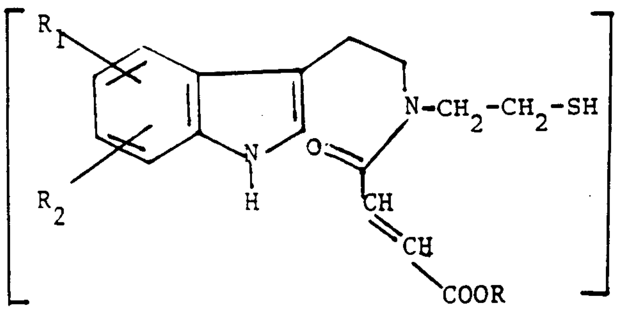

(IV')

qui se cyclise spontanément en composé de formule (V) :

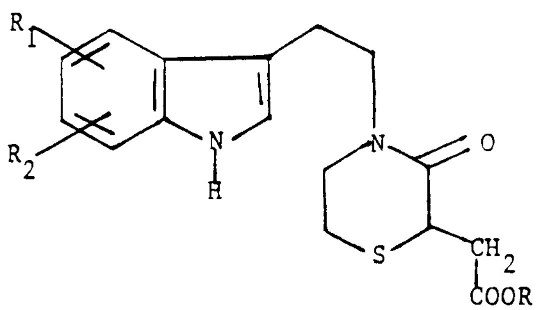

(V)

cyclise le composé de formule (V) en sel de formule (VI) :

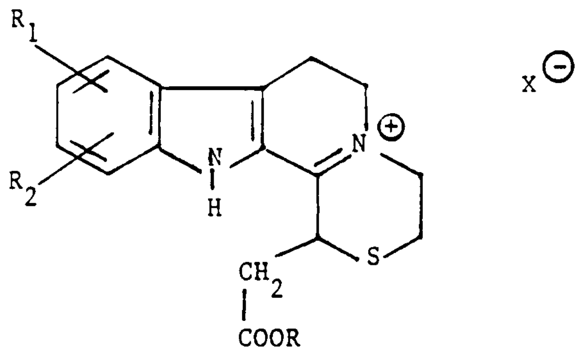

(VI)

dans laquelle $X^-$ représente un contre-ion et que soit l'on réduit le sel de formule (VI) pour obtenir un composé de formule (VII) dans laquelle les deux hydrogènes sont en position cis

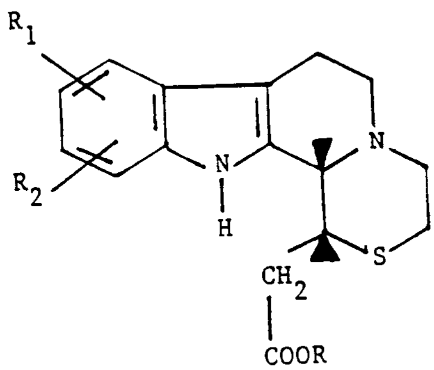

(VII)

produit de formule (VII) que l'on cyclise en milieu basique pour obtenir un composé de formule ($I_{A1}$) correspondant à un composé de formule (I) dans laquelle

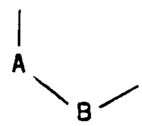

représente

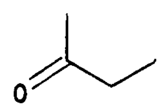

l'atome d'hydrogène en position 3 et l'atome d'hydrogène en position 16 sont en position cis l'un par rapport à l'autre, et n est égal à 0 ; soit l'on cyclise le sel de formule VI en composé de formule (VIII) :

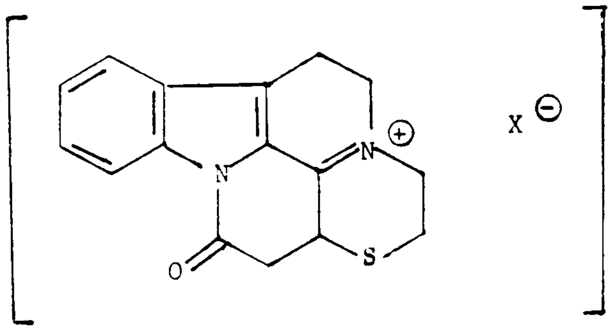

(VIII)

que l'on réduit pour obtenir un composé de formule ($I_{A2}$) correspondant à un composé de formule (I) dans laquelle

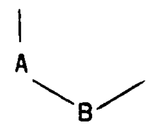

représente

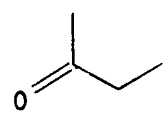

l'atome d'hydrogène en position 3 et l'atome d'hydrogène en position 16 sont en position trans l'un par rapport à l'autre et n est égal à 0 et en ce que lesdits composés de formules ($I_{A1}$) et ($I_{A2}$) sont, si désiré, réduits soit en composés correspondants de formules ($I_{B1}$) et ($I_{B2}$) représentant les composés de formule (I) dans laquelle

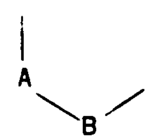

représente

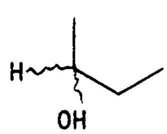

et n est égal à 0 soit en composé de formules ($I_{C1}$) et ($I_{C2}$) représentant les composés de formule (I) dans laquelle

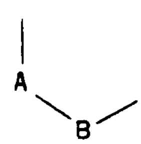

représente

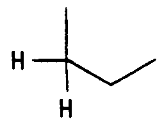

et n est égal à 0 et en ce que, si désiré, on déshydrate les composés de formules $(I_{B1})$ et $(I_{B2})$ pour obtenir les composés de formule $(I_{D1})$ et $(I_{D2})$ représentant les composés de formule (I) dans laquelle

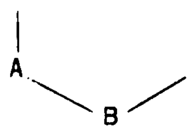

représente

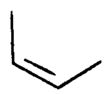

et n est égal a 0, et en ce que l'on soumet si désiré les composés de formules $(I_{A1})$, $(I_{A2})$, $(I_{B1})$, $(I_{B2})$, $I_{C1})$, $(I_{C2})$, $(I_{D1})$ et $(I_{D2})$ à l'action d'un agent d'oxydation pour obtenir les composés correspondants dans lesquels n représente 1 ou 2 et, si désiré, soumet tous les produits de formule (I) obtenus à l'action d'un acide minéral ou organique pour obtenir le sel correspondant.

**5.** Variante du procédé de préparation selon la revendication 4 pour obtenir les composés de formule (I) dans laquelle n est égal à 1 ou 2, caractérisé en ce que l'on soumet les produits de formule $(I_{A1})$ et $(I_{A2})$ dans lesquelles n est égal à 0 à l'action d'agent d'oxydation pour obtenir les produits correspondants dans lesquels n est égal à 1 ou 2, produits que l'on réduit, si désiré, en composés correspondants dans lesquels n est égal à 1 ou 2 et

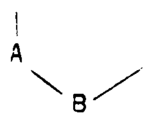

représente

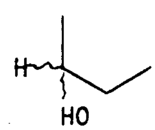

ou

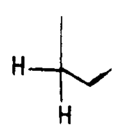

et en ce que, si désiré, l'on déshydrate les produits ainsi obtenus dans lesquels

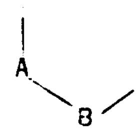

représente

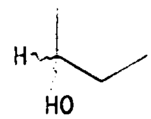

pour obtenir les composés correspondants dans lesquels n est égal à 1 ou 2 et

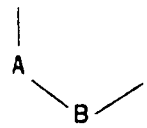

représente

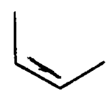

**6.** A titre de médicaments, les composés tels que définis par la formule (I) de la revendication 1, lesdits produits de formule (I) étant sous toutes les formes isomères possibles racémiques ou optiquement actives, ainsi que les sels d'addition avec les acides minéraux ou organiques pharmaceutiquement acceptables.

**7.** A titre de médicaments, les composés tels que définis par les revendications 2 et 3 ainsi que les sels d'addition avec les acides minéraux ou organiques pharmaceutiquement acceptables.

**8.** Les compositions pharmaceutiques contenant à titre de principe actif, l'un au moins des médicaments tels que définis à l'une quelconque des revendications 6 et 7.

**9.** A titre de produits industriels, les composés de formules (IV), (IV'), (V), (VI), (VII), (VIII), tels que définis à la revendication 4.

## Claims

1. Compounds of formula (I):

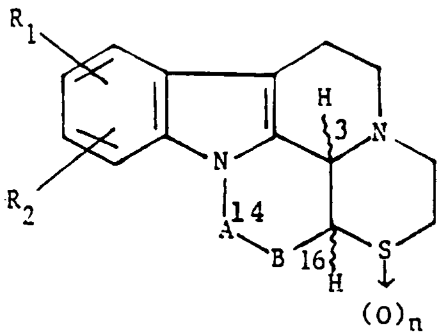

(I)

in which $R_1$ and $R_2$, identical or different, represent a hydrogen atom, a halogen atom, an alkyl or alkoxy radical containing 1 to 5 carbon atoms, a hydroxy, trifluoromethyl or nitro radical and in which n represents 0, 1 or 2 and the group:

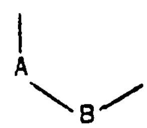

represents: either

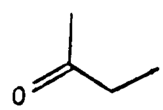

,

or

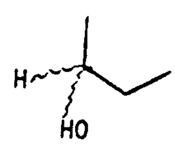

,

or

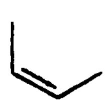

,

or

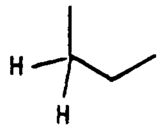

,

the said products of formula (I) being in all the possible racemic or optically active isomer forms, as well as the addition salts with mineral or organic acids of the said products of formula (I).

**2.** Compounds of formula (I) as defined in claim 1, characterized in that $R_1$ and $R_2$ both represent a hydrogen atom and in that the group:

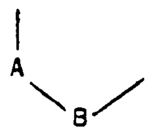

represents either

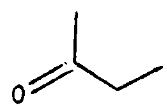

or

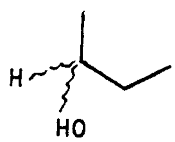

or

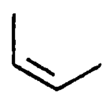

**3.** Compounds of formula (I) as defined in one of claims 1 or 2 and corresponding to the following formulae:

- (±) 20,21-dinor 17-thia eburnamenine 14(15H)-one and its salts with mineral and organic acids, in particular the fumarate,
- (14alpha,16alpha) (±) 14,15-dihydro 20,21-dinor 17-thia eburnamenine-14-ol.

**4.** Preparation process for the compounds of formula (I) as defined in claim 1, characterized in that a compound of formula (II):

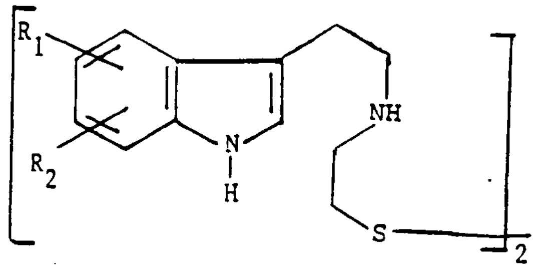

(II)

in which $R_1$ and $R_2$ have the meanings given previously, is subjected to the action of an acid chloride of formula (III) or (III'):

(III') $RO_2C-CH=CH-COCl$

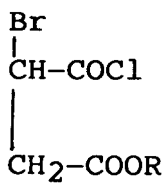

(III)

in which R represents an alkyl radical containing 1 or 2 carbon atoms in order to obtain a compound of formula (IV):

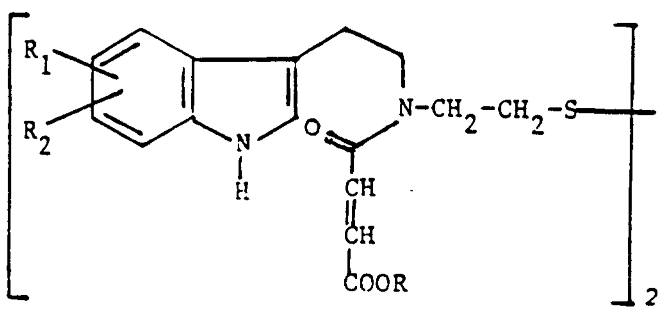

(IV)

the disulphide of formula (IV) is subjected to the action of a reducing agent in order to obtain a compound of formula (IV'):

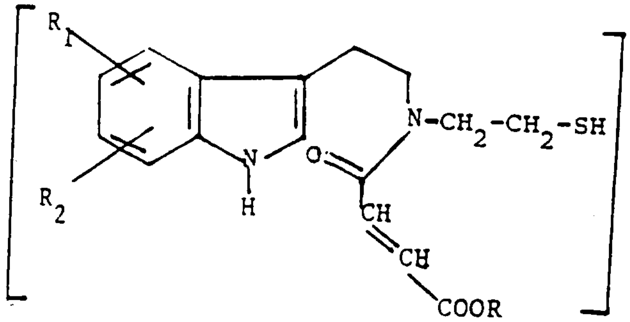

(IV')

which cyclizes spontaneously into a compound of formula (V):

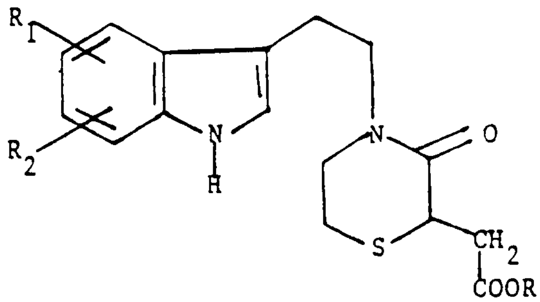

(V)

the compound of formula (V) is cyclized into a salt of formula (VI):

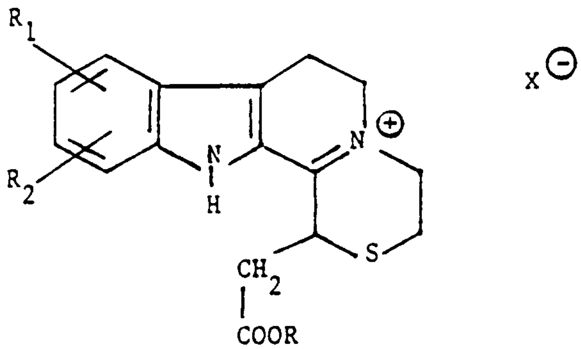

(VI)

in which $X^-$ represents a counter ion and <u>either</u> the salt of formula (VI) is reduced in order to obtain a compound of formula (VII) in which the two hydrogens are in cis position:

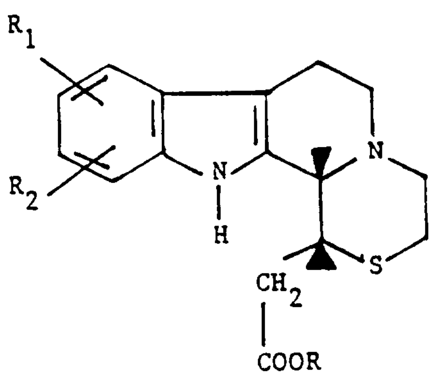

(VII)

which product of formula (VII) is cyclized in a basic medium in order to obtain a compound of formula ($I_{A1}$) corresponding to a compound of formula (I) in which

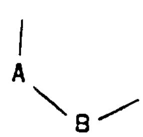

represents

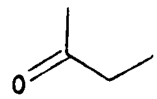

the hydrogen atom in position 3 and the hydrogen atom in position 16 are in cis position relative to each other, and n is equal to 0;
or the salt of formula (VI) is cyclized into a compound of formula (VIII):

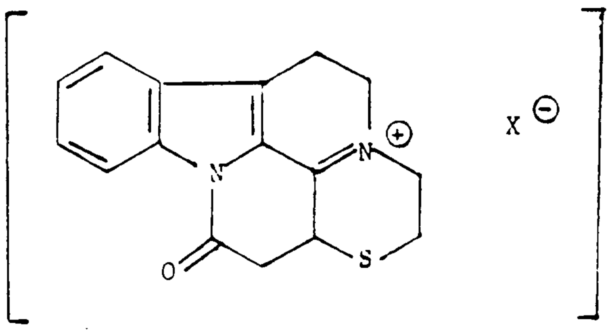

(VIII)

which is reduced in order to obtain a compound of formula ($I_{A2}$) corresponding to a compound of formula (I) in which

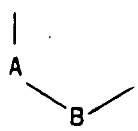

represents

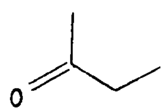

the hydrogen atom in position 3 and the hydrogen atom in position 16 are in trans position relative to each other and n is equal to 0, and in that the said compounds of formulae ($I_{A1}$) and ($I_{A2}$) are, if desired, reduced either into corresponding compounds of formulae ($I_{B1}$) and ($I_{B2}$) representing the compounds of formula (I) in which

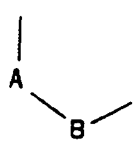

represents

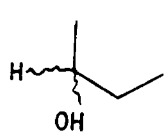

and n is equal to 0 or into compounds of formulae ($I_{C1}$) and ($I_{C2}$) representing the compounds of formula (I) in which

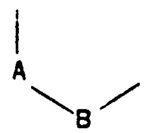

represents

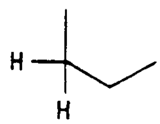

and n is equal to 0, and in that, if desired, the compounds of formulae ($I_{B1}$) and ($I_{B2}$) are dehydrated in order to obtain the compounds of formula ($I_{D1}$) and ($I_{D2}$) representing the compounds of formula (I) in which

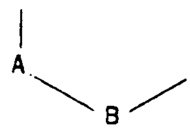

represents

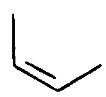

and n is equal to 0, and in that, if desired, the compounds of formulae ($I_{A1}$),($I_{A2}$), ($I_{B1}$), ($I_{B2}$), ($I_{C1}$), ($I_{C2}$), ($I_{D1}$) and ($I_{D2}$) are subjected to the action of an oxidation agent in order to obtain the corresponding compounds in which n represents 1 or 2 and, if desired, all the products of formula (I) obtained are subjected to the action of a mineral or organic acid in order to obtain the corresponding salt.

**5.** Variation of the preparation process according to claim 4 in order to obtain the compounds of formula (I) in which n is equal to 1 or 2, characterized in that the products of formula ($I_{A1}$) and ($I_{A2}$) in which n is equal to 0 are subjected to the action of an oxidation agent in order to obtain the corresponding products in which n is equal to 1 or 2, which products are reduced, if desired, into the corresponding compounds in which n is equal to 1 or 2 and

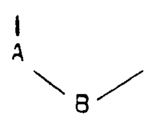

represents

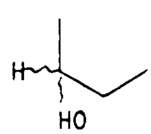

or

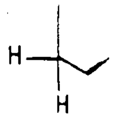

and in that, if desired, the products thus obtained in which

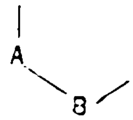

represents

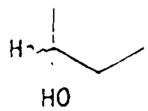

are dehydrated in order to obtain the corresponding compounds in which n is equal to 1 or 2 and

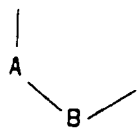

represents

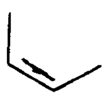

**6.** As medicaments, the compounds as defined by formula (I) of claim 1, the said products of formula (I) being in all the possible racemic and optically active isomer forms, as well as the pharmaceutically acceptable addition salts with mineral or organic acids.

**7.** As medicaments, the compounds as defined by claims 2 and 3 as well as the pharmaceutically acceptable addition salts with mineral or organic acids.

**8.** The pharmaceutical compositions containing as active ingredient at least one of the medicaments as defined in any one of claims 6 and 7.

**9.** As industrial products, the compounds of formulae (IV), (IV'), (V), (VI), (VII), (VIII), as defined in claim 4.

## Patentansprüche

**1.** Verbindungen der Formel (I):

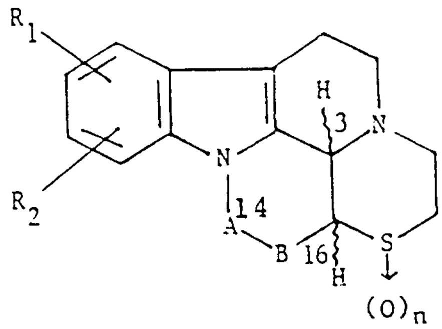

(I)

in der $R_1$ und $R_2$, die gleich oder verschieden sein können, ein Wasserstoffatom, ein Halogenatom, einen Alkyl- oder Alkoxyrest mit 1 bis 5 Kohlenstoffatomen, einen Hydroxy-, Trifluormethyl- oder Nitrorest darstellen und in der n 0, 1 oder 2 darstellt und die Gruppe:

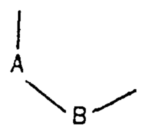

entweder

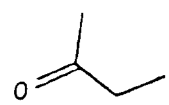

oder

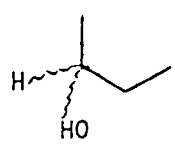

oder

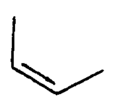

oder

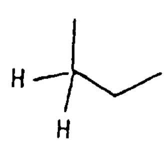

darstellt, wobei die Produkte der Formel (I) in allen möglichen racemischen oder optisch aktiven isomeren Formen vorliegen, sowie die Additionssalze mit Mineral- oder organischen Säuren der Produkte der Formel (I).

**2.** Verbindungen der Formel (I), wie in Anspruch 1 definiert, dadurch gekennzeichnet, daß $R_1$ und $R_2$ alle beide ein Wasserstoffatom darstellen und daß die Gruppe:

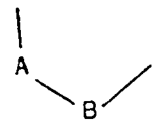

entweder

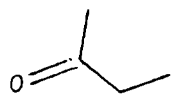

oder

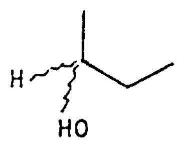

oder

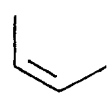

darstellt.

**3.** Verbindungen der Formel (I), wie in einem der Ansprüche 1 oder 2 definiert und den folgenden Formeln entsprechend:
(±)-20,21-Dinor-17-thiaeburnamenin-14(15H)-on und seine Salze mit Mineral- oder organischen Säuren, insbesondere das Fumarat,
(14alpha,16alpha) (±)-14,15-Dihydro-20,21-dinor-17-thiaeburnamenin-14-ol.

**4.** Verfahren zur Herstellung der Verbindungen der Formel (I), wie in Anspruch 1 definiert, dadurch gekennzeichnet, daß man eine Verbindung der Formel (II):

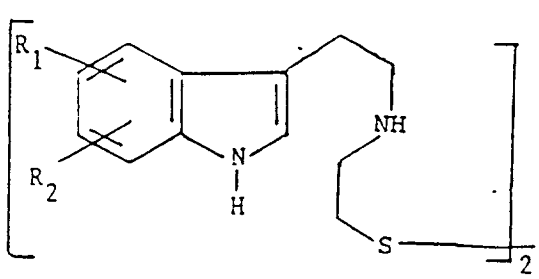

(II)

in der $R_1$ und $R_2$ die vorstehend angegebenen Bedeutungen haben, der Einwirkung eines Säurechlorids der Formel (III) oder (III'):

(III') $RO_2C\text{-}CH=CH\text{-}COCl$ ,

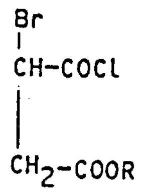

(III)

in der R einen Alkylrest mit 1 oder 2 Kohlenstoffatomen darstellt, unterzieht, um eine Verbindung der Formel (IV):

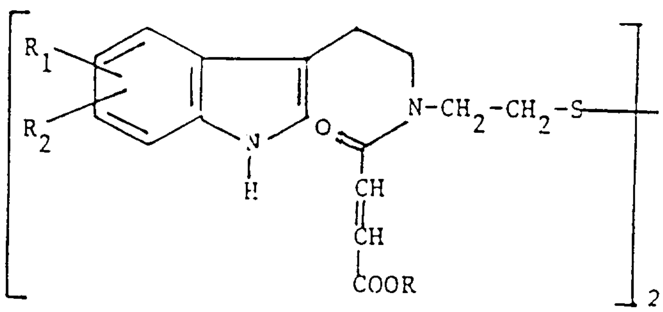

(IV)

zu erhalten,
das Disulfid der Formel (IV) der Einwirkung eines Reduktionsmittels unterzieht, um eine Verbindung der Formel (IV'):

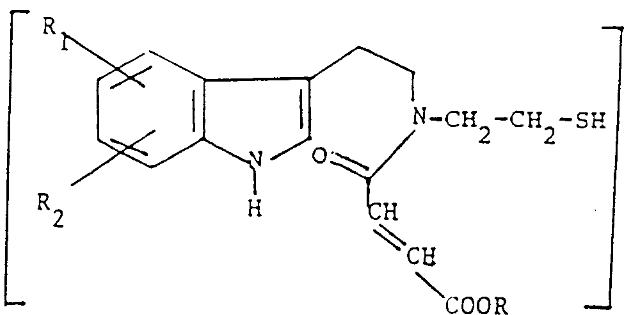

(IV')

zu erhalten, die spontan zur Verbindung der Formel (V):

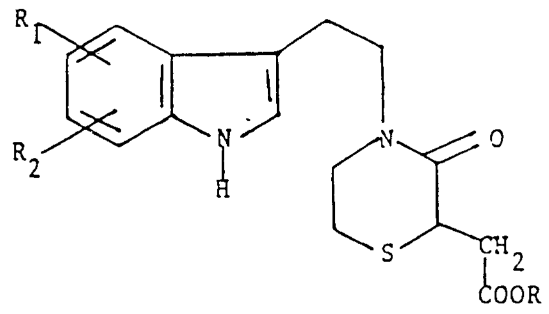

(V)

cyclisiert,
die Verbindung der Formel (V) zum Salz der Formel (VI):

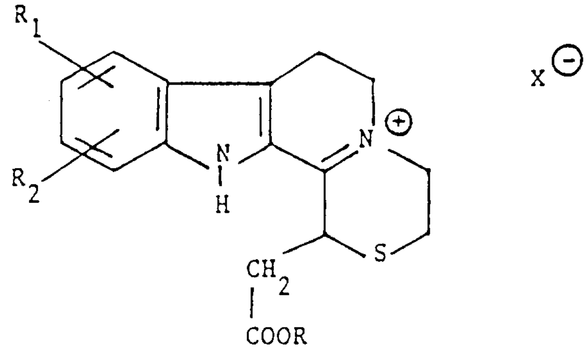

(VI)

in der X⁻ ein Gegenion darstellt, cyclisiert und daß man entweder das Salz der Formel (VI) reduziert, um eine Verbindung der Formel (VII) zu erhalten, in der die beiden Wasserstoffe in cis-Stellung sind

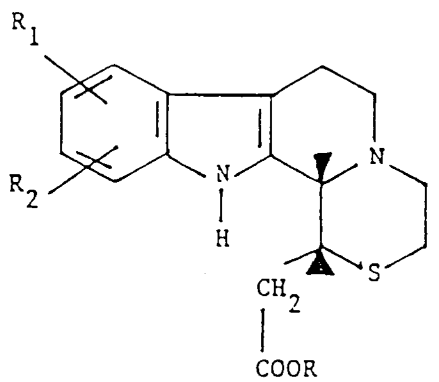

(VII)

man das Produkt der Formel (VII) im basischen Milieu cyclisiert, um eine Verbindung der Formel ($I_{A1}$) zu erhalten, die einer Verbindung der Formel (I) entspricht, in welcher

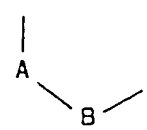

für

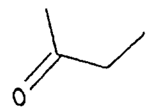

steht, das Wasserstoffatom in Stellung 3 und das Wasserstoffatom in Stellung 16 in cis-Stellung zueinander stehen und n gleich 0 ist; oder daß man das Salz der Formel VI zu einer Verbindung der Formel (VIII):

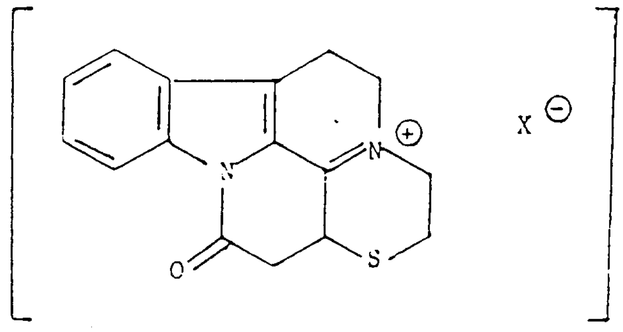

(VIII)

cyclisiert, die man reduziert, um eine Verbindung der Formel ($I_{A2}$) zu erhalten, die einer Verbindung der Formel (I) entspricht, in der

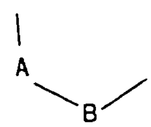

für

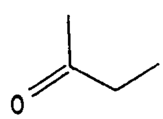

steht, das Wasserstoffatom in Stellung 3 und das Wasserstoffatom in Stellung 16 in trans-Stellung zueinander stehen und n gleich 0 ist, und

daß die Verbindungen der Formeln ($I_{A1}$) und ($I_{A2}$), falls gewünscht, entweder zu Verbindungen, die den Formeln ($I_{B1}$) und ($I_{B2}$) entsprechen, welche die Verbindungen der Formel (I) darstellen, in der

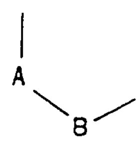

für

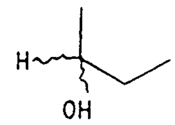

steht und n gleich 0 ist,
oder zu einer Verbindung der Formeln ($I_{C1}$) und ($I_{C2}$), welche die Verbindungen der Formel (I) darstellen, in der

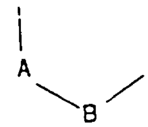

für

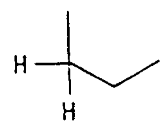

steht und n gleich 0 ist, reduziert werden,
und daß man, falls gewünscht, die Verbindungen der Formeln ($I_{B1}$) und ($I_{B2}$) dehydratisiert, um die Verbindungen der Formeln ($I_{D1}$) und ($I_{D2}$) zu erhalten, welche die Verbindungen der Formel (I) darstellen, in der

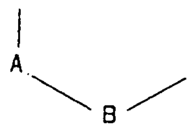

für

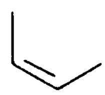

steht und n gleich 0 ist,
und daß man, falls gewünscht, die Verbindungen der Formeln ($I_{A1}$), ($I_{A2}$), ($I_{B1}$), ($I_{B2}$), ($I_{C1}$), ($I_{C2}$), ($I_{D1}$) und ($I_{D2}$) der Einwirkung eines Oxidationsmittels unterzieht, um die entsprechenden Verbindungen zu erhalten, in denen n 1 oder 2 darstellt,
und, falls gewünscht, alle erhaltenen Produkte der Formel (I) der Einwirkung einer Mineral- oder organischen Säure unterzieht, um das entsprechende Salz zu erhalten.

**5.** Variante des Herstellungsverfahrens nach Anspruch 4, um die Verbindungen der Formel (I) zu erhalten, in der n gleich 1 oder 2 ist, dadurch gekennzeichnet, daß man die Produkte der Formel ($I_{A1}$) und ($I_{A2}$), in denen n gleich 0 ist, der Einwirkung eines Oxidationsmittels unterzieht, um die entsprechenden Produkte, in denen n gleich 1 oder 2 ist, zu erhalten, man die Produkte, falls gewünscht, zu den entsprechenden Verbindungen, in denen n gleich 1 oder 2 ist und

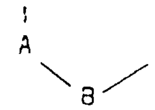

für

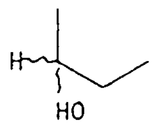

oder

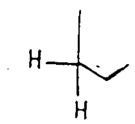

steht, reduziert und daß man, falls gewünscht, die so erhaltenen Produkte, in denen

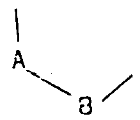

für

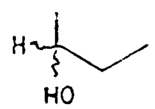

steht, dehydratisiert, um die entsprechenden Verbindungen zu erhalten, in denen n gleich 1 oder 2 ist und

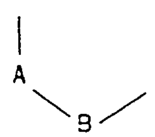

für

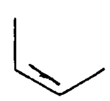

steht.

**6.** Verbindungen, wie durch die Formel (I) des Anspruchs 1 definiert, wobei die Produkte der Formel (I) in allen möglichen racemischen oder optisch aktiven isomeren Formen vorliegen, sowie die pharmazeutisch annehmbaren Additionssalze mit Mineral- oder organischen Säuren als Arzneimittel.

**7.** Verbindungen, wie durch die Ansprüche 2 und 3 definiert, sowie die pharmazeutisch annehmbaren Additionssalze mit Mineral- oder organischen Säuren als Arzneimittel.

**8.** Pharmazeutische Zusammensetzungen, die als Wirkstoff mindestens eines der wie in irgendeinem der Ansprüche 6 und 7 definierten Arzneimittel enthalten.

**9.** Verbindungen der Formeln (IV), (IV'), (V), (VI), (VII), (VIII), wie in Anspruch 4 definiert, als Industrieprodukte.